# EUROPEAN PATENT SPECIFICATION

(11) **EP 2 168 629 B1**
(45) Date of publication and mention of the grant of the patent: **08.01.2014**
(21) Application number: 09170251.4
(22) Date of filing: 15.09.2009
(51) Int. Cl.: A61N 1/04, A61N 1/32, A61N 1/30

(54) **System of prepping skin prior to electrode application**
System zur Präparierung von Haut vor der Elektrodenanwendung
Système pour la préparation d'une peau avant l'application d'électrodes

(30) Priority: 25.09.2008 US 237768
(43) Date of publication of application: 31.03.2010
(73) Proprietor: Covidien LP, Mansfield, MA 02048 (US)
(72) Inventor: Copp-Howland, Warren W., Chicopee, MA 01013 (US); Tremblay, Kathleen, Mansfield, MA 01085 (US)
(74) Representative: Gray, James

(56) References cited:
- WO-A-97/24060
- WO-A-98/49955
- WO-A-2005/012549
- FR-A- 2 811 524
- GB-A- 898 299
- US-A- 4 474 570
- US-A- 6 161 546
- US-A1- 2006 276 741
- US-A1- 2007 074 590
- US-A1- 2007 183 936
- US-B1- 6 341 230

## Description

### BACKGROUND

### 1. Technical Field

The present invention relates to surgical/medical procedures and, more particularly, to electrodes with conductive compositions containing exfoliants to aid in preparing the surface of the skin of a patient prior to an application of an electrode thereto.

### 2. Background of Related Art

Electrodes (e.g., diagnostic, therapeutic and/or monitoring type) are used to transmit electrical signals or currents between the body of a patient and external or remote equipment (e.g., diagnostic, therapeutic and/or monitoring type). Such electrodes typically include a conductive composition adherable to or otherwise contactable with, the skin of the patient, and a conductor, which is electrically connected to the conductive composition and to the external equipment. A known electrode is shown in US2006/0276741.

The degree of effectiveness and/or responsiveness of the electrode is related to the characteristics of the under-lying skin of the patient. The skin naturally has a high impedance level. Typically, the higher the impedance, the more difficult it is for the electrical signal to penetrate the stratum corneum. The more difficult it is for the electrical signal to traverse the stratum corneum the more time is required for the electrical signal to be delivered and received and the quality of the electrical signal is reduced.

In practice, clinicians may prepare the skin by removing dead layers of stratum corneum prior to placing an electrode on the skin of a patient. Common procedures include the use of alcohol swabs, cleansing the skin with water, soaps, or detergents, and using an abrasive agent like pummus.

Accordingly, it is desirable to treat the skin of a patient in a manner which would reduce the impedance thereof, where improved signal acquisition and/or energy delivery may be achieved.

A further need exists for an electrode which reduces impedance.

Accordingly, a need exists for a system of treating the skin of a patient, prior to and/or during application of an electrode thereto, in order to reduce the level of impedance of the skin and thereby improve signal acquisition and/or energy delivery.

### SUMMARY

The present invention relates to biomedical electrodes of preparing the surface of the skin of a patient prior to an application of an electrode thereto.

According to the present invention, there is provided an electrode according to claim 1. The electrode is for selective attachment to the skin of a subject, wherein the skin of the subject has an impedance. The electrode includes a conductive member defining a first side and a second side; a conductive composition disposed on the first side of the conductive member, wherein the conductive composition includes a therapeutically effective quantity of an exfoliant; and an electrical lead in electrical communication with the conductive member. In use, the conductive composition reduces a level of the impedance of the skin of the subject.

The conductive composition may be selectively adherable to the skin of a subject.

The exfoliant may be an alphahydroxy acid. The alphahydroxy acid may have a concentration of 1-100%. The conductive composition may include approximately 0.01-10% of alphahydroxy acid.

The electrode may include a backing member disposed on the second side of the conductive member. The electrode may include a release liner selectively, removably adhered to a surface of the conductive composition.

The conductive composition may be a hydrogel.

The electrode may include a reinforcement member supporting the conductive composition. The electrode may include a coating of silver provided on at least a portion of at least one of the first and second sides of the conductive member.

The electrical lead may be a pig-tail style lead, snap style lead or tab style lead.

The exfoliant may be an alphahydroxy acid. The alphahydroxy acid may have a concentration of 1-100%. The conductive composition may include about 0.01-10% of alphahydroxy acid.

### DETAILED DESCRIPTION OF THE DRAWINGS

The accompanying drawings, which are incorporated in and constitute a part of this specification, illustrate different aspects of the invention and serve to explain the principles of the invention, wherein:

FIG. 1 is a schematic, perspective view of an exemplary leadwire style electrode of the present disclosure, shown with layers separated;

FIG. 2 is a cross-sectional view, of the electrode of FIG. 1;

FIG. 3 is a cross-sectional view, of an alternate leadwire style electrode, according to an aspect of the present invention;

FIG. 4 is a cross-sectional view of an exemplary snap-type electrode of the present disclosure; and

FIG. 5 is a cross-sectional view, of a tab style electrode, according to an aspect of the present invention.

### DETAILED DESCRIPTION

Aspects of the presently disclosed system will now be described in detail with reference to the drawing figures wherein like reference numerals identify similar or identical elements.

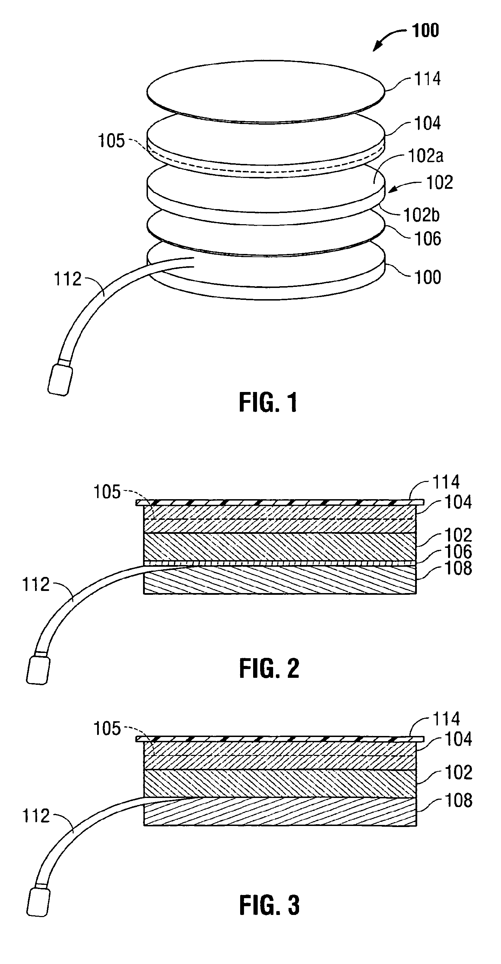

Referring initially to FIGS. 1 and 2, an electrode in accordance with an aspect of the present invention is generally designated as electrode 100. Electrode 100 includes a conductive member 102 defining a first or skin side 102a relative to a subject and a second side 102b, opposite first side 102a. Conductive member 102 may be made from a conductive carbon, aluminum, tin or any other suitable conductive material. As an alternative, conductive member 102 may comprise a conductive plastic material. Conductive member 102 may include silver (Ag) or silver/silver-chloride (Ag/AgCl) material deposited on at least a portion of first side 102a or second side 102b. Either first side 102a or second side 102b may also have a coating of silver (Ag) or silver/silver-chloride (Ag/AgCl) composition or ink 106 on either first side 102a or second side 102b thereof.

Alternatively, as seen in FIG. 3, electrode 100 may be free of the coating of silver (Ag) or silver/silver-chloride (Ag/AgCl) composition or ink 106.

Turning back to FIGS. 1 and 2, electrode 100 further includes a conductive composition 104 disposed adjacent first side 102a of the conductive member 102 for application/adhesion to or contact with the skin of the subject. Conductive composition 104 may be made from, for example, but not limited to, Promeon RG-63B hydrogel (TycoHealthcare Group LP d/b/a Covidien). As seen in FIGS. 1 and 2 conductive composition 104 may incorporate a reinforcement member. The reinforcement member may be a woven or non-woven cloth or gauze material (e.g., scrim) 105 embedded therewithin or supporting the structure of the hydrogel. The reinforcement member may be made from a conductive material. The conductive composition 104 may be any different commercially available conductive hydrogel. Conductive composition 104 is generally hydrophilic.

Conductive composition 104 has a therapeutically effective amount of an exfoliant incorporated therein. For example, conductive composition 104 may be provided with an alphahydroxy acid (AHA) incorporated therein. Conductive composition 104 may alternatively or further be provided with glycolic acid, citric acid, and/or boric acid incorporated therein. The exfoliant is added to conductive composition 104 in an amount sufficient to exfoliate the skin of the subject without or with minimal subsequent skin irritation to the patient. In an example, the exfoliant has a concentration of about 0.01-5%.

By providing conductive composition 104 with a therapeutically effective quantity of an exfoliant, upon application of electrode 100 to the skin of the subject, at least some dead skin cells in the stratum corneum may be cleaned away or otherwise more easily penetrated. By reducing the thickness of the dead skin cells in the stratum corneum or by removing the dead skin cells in the stratum corneum, an impedance level of the skin may be reduced. As compared to the untreated skin of a subject, treated skin of the subject exhibits a reduced level of impedance, thereby reducing a relative time it takes for electrode 100 to acquire a signal for a sensing procedure and/or reducing a level of resistance encountered by electrode 100 for a stimulating procedure.

A first side release liner 114 is releasably secured to conductive composition 104. Release liner 114 can be made from a film or paper substrate having a release coating on one or both sides, such as, for example silicone. Release liner 114 protects and/or preserves conductive composition 104 (e.g., the hydrogel) and is removed prior to application on the skin of the subject. Release liner 114 may be applied to conductive composition 104 after use of electrode 100 to preserve the conductive composition 104 for subsequent use.

Release liner 114 may be a release paper or film of a waxed or coated plastic, such as a silicone coated polyethylene terephthalate film, which may be used to protect electrode 100 before application of the electrode to the skin of the subject.

Electrode 100 may further include a backing member 108 disposed adjacent second side 102b of conductive member 102. Backing member 108 may overlie silver coating 106. Backing member 108 is fabricated from a non-conductive material such as a cloth, fabric, plastic material or the like.

Electrode 100 further includes an electrical lead or lead wire 112, e.g., a lead wire having a pig tail configuration that is in electrical communication with at least conductive member 102 and a power supply (not shown). Electrical communication extends from lead wire 112 through the conductive member 102 (and silver coating 106) and through conductive composition 104 to the subject.

In use, release liner 114 is removed from electrode 100. Electrode 100 is then applied to the skin of the subject, such that conductive composition 104 is adhered to the skin of the subject. Electrode 100 is then electrically connected to external medical equipment (not shown) by any connection means well known in the art, such as, for example, via lead wire 112. Other leads are well known in the art, including and not limited to snap style, tab style, rivet-post style, etc. Electrode 100 may, by way of example, be connected to a transcutaneous electrical nerve stimulation (TENS) unit by means known to one having skill in the art.

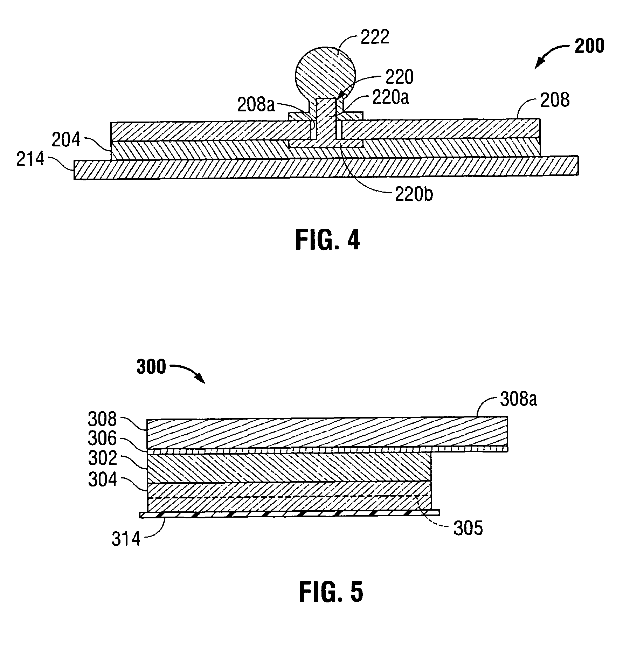

Turning now to FIG. 4, a cross-section of a snap-style electrode 200, in accordance with the present invention is shown. Electrode 200 includes a non-conductive backing member 208 having an opening 208a formed therein, and a conductive composition 204 supported thereon.

Electrode 200 includes an eyelet 220 having a stem 220a protruding through opening 208a, in a direction away from the skin of a subject, and a base 220b disposed or embedded in conductive composition 204 and being on the same side as the skin of the subject. Stem 208a of eyelet 208 is covered by a snap 222 which is securely connected thereto. Together snap 222 and eyelet 220 define at least part of a conductive pathway to provide an electrical connection between conductive composition 204 and the external medical equipment (not shown).

A release liner 214 may be provided to protect conductive composition 204 prior to use.

Similar to electrode 100, conductive composition 204 of electrode 200 includes a quantity of a therapeutically effective amount of an exfoliant therein. For example, conductive composition 204 may be provided with an alphahydroxy acid (AHA) incorporated therein. Conductive composition 204 may alternatively or further be provided with acetic, citric and/or boric acid incorporated therein. The exfoliant is added to conductive composition 204 in an amount sufficient to exfoliate the skin of the subject without or with minimal subsequent skin irritation to the patient. In an example, the exfoliant has a concentration of about 0.01-5%.

Referring to FIG. 5, a cross-section of a tab-style electrode 300, in accordance with the present invention is shown. Electrode 300 includes a non-conductive backing member 308, and a conductive composition 304 supported thereon. Backing member 308 includes a portion 308a extending beyond a perimeter or edge of conductive member 202 thereby defining a tab. As seen in FIG. 5, silver coating 306 may extend onto the surface of tab portion 308a of backing member 308. A release liner 314 may be provided to protect conductive composition 304 prior to use.

Similar to electrodes 100 and 200, conductive composition 304 of electrode 300 includes a quantity of a therapeutically effective amount of an exfoliant therein. For example, conductive composition 304 may be provided with an alphahydroxy acid (AHA) incorporated therein. Conductive composition 304 may alternatively or further be provided with glycolic acid, citric acid, and/or boric acid incorporated therein. The exfoliant is added to conductive composition 304 in an amount sufficient to exfoliate the skin of the subject without or with minimal subsequent skin irritation to the patient. In an example, the exfoliant has a concentration of about 0.01-5%.

It will be appreciated that various examples of the invention and other features and functions, or alternatives thereof, may be desirably combined into many other different systems or applications. Also that various presently unforeseen or unanticipated alternatives, modifications, variations or improvements therein may be subsequently made by those skilled in the art which are also intended to be encompassed by the following claims. Unless specifically recited in a claim, components of claims should not be implied or imported from the specification or any other claims as to any particular order, number, position, size, or material.

## Claims

1. An electrode (100, 200, 300) for selective attachment to the skin of a subject, wherein the skin of the subject has an impedance, the electrode (100, 200, 300) comprising:
a conductive member (102, 302) defining a first side (102a) and a second side (102b);
a conductive composition (104, 204, 304) disposed on the first side (102a) of the conductive member (102); and
an electrical lead (112, 222) in electrical communication with the conductive member (102,302),
**characterised in that**;
the conductive composition (104, 304) includes a therapeutically effective quantity of an exfoliant and the quantity of the exfoliant included in the conductive composition (104, 204, 304) is in an amount sufficient to exfoliate the skin of the subject without or with minimal subsequent skin irritation to the patient, and **in that** the exfoliant is alphahydroxy acid or boric acid.

2. The electrode (100, 200, 300) according to claim 1, wherein the alphahydroxy acid has a concentration of 1-100%.

3. The electrode (100, 200, 300) according to claim 1, wherein the conductive composition (104, 204, 304) includes about 0.01-10% of alphahydroxy acid.

4. The electrode (100) according to any preceding claim, further comprising a backing member (114) disposed on the second side of the conductive member (102).

5. The electrode (300) according to any preceding claim, further comprising a release liner (314) selectively, removably adhered to a surface of the conductive composition (304).

6. The electrode (100, 200, 300) according to any preceding claim, wherein the conductive composition is a hydrogel.

7. The electrode (100, 300) according to any preceding claim, further comprising a reinforcement member (105, 305) supporting the conductive composition (104, 304).

8. The electrode (100) according to any preceding claim, further comprising at least one of a coating of silver and silver-chloride on at least a portion of at least one of the first and second sides (102, 102b) of the conductive member (104).

9. The electrode (100, 200, 300) according to any preceding claim, wherein the electrical lead is one of a pig-tail style electrical lead (112), a snap style electrical lead (222) and a tab style electrical lead (308a).

## Patentansprüche

1. Elektrode (100, 200, 300) für das selektive Anbringen an der Haut einer Person, wobei die Haut der Person eine Impedanz aufweist, wobei die Elektrode(100, 200, 300) Folgendes umfasst:
ein leitendes Glied (102, 302), das eine erste Seite (102a) und eine zweite Seite (102b) definiert;
eine leitende Zusammensetzung (104, 204, 304), die auf der ersten Seite (102a) des leitenden Glieds (102) angeordnet ist; und
eine Stromleitung (112, 222) in elektrischer Kommunikation mit dem leitenden Glied (102, 302),
**dadurch gekennzeichnet, dass**
die leitende Zusammensetzung (104, 304) eine therapeutisch effektive Menge eines Exfoliant-Mittels enthält und die Menge des in der leitenden Zusammensetzung (104, 204, 304) enthaltene Menge des Exfoliant-Mittels eine Menge ist, die ausreicht, um die Haut der Person ohne oder mit minimaler nachfolgender Hautreizung für den Patienten zu exfolieren, und dass das Exfoliant-Mittel Alpha-Hydroxy-Säure oder Borsäure ist.

2. Elektrode (100, 200, 300) nach Anspruch 1, wobei die Alpha-Hydroxy-Säure eine Konzentration von 1-100% aufweist.

3. Elektrode (100, 200, 300) nach Anspruch 1, wobei die leitende Zusammensetzung (104, 204, 304) etwa 0,01-10% Alpha-Hydroxy-Säure enthält.

4. Elektrode (100) nach einem vorhergehenden Anspruch, weiterhin umfassend ein auf der zweiten Seite des leitenden Glieds (102) angeordnetes Unterlagenglied (114).

5. Elektrode (300) nach einem vorhergehenden Anspruch, weiterhin umfassend eine Trennzwischenlage (314), die selektiv entfernbar an einer Oberfläche der leitenden Zusammensetzung (304) haftet.

6. Elektrode (100, 200, 300) nach einem vorhergehenden Anspruch, wobei die leitende Zusammensetzung ein Hydrogel ist.

7. Elektrode (100, 300) nach einem vorhergehenden Anspruch, weiterhin umfassend ein Verstärkungsglied (105, 305), das die leitende Zusammensetzung (104, 304) stützt.

8. Elektrode (100) nach einem vorhergehenden Anspruch, weiterhin umfassend mindestens eine Beschichtung aus Silber oder Silberchlorid auf mindestens einem Abschnitt mindestens einer der ersten und zweiten Seite (102, 102b) des leitenden Glieds.

9. Elektrode (100, 200, 300) nach einem vorhergehenden Anspruch, wobei eine Stromleitung eine Stromleitung vom Anschlusstyp (112), eine Stromleitung vom Schnapptyp (222) oder eine Stromleitung vom Fahnentyp (308a) ist.

## Revendications

1. Electrode (100, 200, 300) pour une fixation sélective à la peau d'un sujet, dans laquelle la peau du sujet a une impédance, l'électrode (100, 200, 300) comprenant :
un élément conducteur (102, 302) définissant un premier côté (102a) et un deuxième côté (102b) ;
une composition conductrice (104, 204, 304) disposée sur le premier côté (102a) de l'élément conducteur (102) ; et
un conducteur électrique (112, 222) en communication électrique avec l'élément conducteur (102, 302),
**caractérisée en ce que**
la composition conductrice (104, 304) comprend une quantité thérapeutiquement efficace d'un exfoliant et la quantité de l'exfoliant inclus dans la composition conductrice (104, 204, 304) est une quantité suffisante pour exfolier la peau du sujet sans irritation de la peau du patient ou avec une irritation subséquente minimale, et **en ce que** l'exfoliant est un acide α-hydroxylé ou un acide borique.

2. Electrode (100, 200, 300) selon la revendication 1, dans laquelle l'acide α-hydroxylé a une concentration de 1 à 100 %.

3. Electrode (100, 200, 300) selon la revendication 1, dans laquelle la composition conductrice (104, 204, 304) comprend environ 0,01 à 10 % d'acide α-hydroxylé.

4. Electrode (100) selon l'une quelconque des revendications précédentes, comprenant en outre un élément arrière (114) disposé sur le deuxième côté de l'élément conducteur (102).

5. Electrode (300) selon l'une quelconque des revendications précédentes, comprenant en outre une bande antiadhésive (314) collée de manière sélective et de manière amovible à une surface de la composition conductrice (304).

6. Electrode (100, 200, 300) selon l'une quelconque des revendications précédentes, dans laquelle la composition conductrice est un hydrogel.

7. Electrode (100, 300) selon l'une quelconque des revendications précédentes, comprenant en outre un élément de renforcement (105, 305) supportant la composition conductrice (104, 304).

8. Electrode (100) selon l'une quelconque des revendications précédentes, comprenant en outre au moins l'un d'un revêtement en argent et d'un revêtement en chlorure d'argent sur au moins une partie d'au moins l'un des premier et deuxième côtés (102, 102b) de l'élément conducteur (104).

9. Electrode (100, 200, 300) selon l'une quelconque des revendications précédentes, dans laquelle le conducteur électrique est l'un d'un conducteur électrique du style queue de cochon (112), d'un conducteur électrique du style à encliqueter (222) et d'un conducteur électrique du style languette (308a).
